# EUROPÄISCHE PATENTSCHRIFT

(11) **EP 2 179 712 B1**
(45) Veröffentlichungstag und Bekanntmachung des Hinweises auf die Patenterteilung: **10.07.2013**
(21) Anmeldenummer: 09013316.6
(22) Anmeldetag: 21.10.2009
(51) Int. Cl.: A61F 5/01

(54) **Orthese zum gestreckten Halten flexibler knöcherner Strukturen**
Orthosis for elongated holding of flexible bony structures
Orthèse de maintien étendu de structures osseuses flexibles

(30) Priorität: 21.10.2008 DE 102008052517; 21.10.2008 DE 102008052369
(43) Veröffentlichungstag der Anmeldung: 28.04.2010
(73) Patentinhaber: Pohlig GmbH, 83278 Traunstein (DE)
(72) Erfinder: Pohlig, Kurt, 83278 Traunstein (DE)
(74) Vertreter: Paustian, Othmar

(56) Entgegenhaltungen:
- EP-A- 1 086 671
- WO-A-91/13604
- WO-A-97/13487
- DE-A1-102007 002 532
- US-A- 2 934 064
- US-A- 4 099 525
- US-A1- 2005 273 028

## Beschreibung

Die Erfindung betrifft eine Orthese nach dem Oberbegriff von Anspruch 1.

Vor allem für Behandlungsverfahren und Operationstechniken im Zusammenhang mit flexiblen knöchernen Strukturen, etwa erkrankten, abgenutzten oder operierten Gelenken, kann es wünschenswert sein, diese knöchernen Strukturen gezielt entlastet zuhalten oder sogar auseinanderziehen zu können.

Bekannt ist zum Beispiel der "Fixateur externe" (frz.; "äußerer Festhalter") zum Ruhigstellen eines Knochenbruches oder zur Knochenverlängerung. Hier werden Pins auf beiden Seiten des Knochenbruchs beziehungsweise der auseinanderzuziehenden knöchernen Struktur durch die Haut und das sonstige Gewebe in den Knochen getrieben und dort verankert. Üblicherweise werden die Pins über Stangen außerhalb des Körpers fest miteinander verbunden. Soll ein Knochen verlängert werden, so wird sukzessive eine Callusbildung immer wieder angeregt. Dabei werden die Pins über die Stangen sukzessive zunehmend auseinander gedrückt. Die Anwendung eines Fixateur externe ist recht belastend.

Eine gattungsgemäße Orthese ist aus der DE 10 2007 002532 A1 bekannt. Die Länge der Orthese kann dem Längenwachstum eines solch eine Orthese tragenden Kindes angepasst werden. Es wird eine Einrichtung zur Längsverschiebung vorgeschlagen, mit der quer zur Schieberichtung gerichtete Relativkräfte und/oder Verdrehkräfte aufgenommen werden können und die eine Arretiervorrichtung zur Aufnahme von Relativkräften in Schieberichtung aufweist. Es wird insbesondere vorgeschlagen, die Arretiervorrichtung als Klemm- oder Rasteinrichtung auszuführen. Die Einstellvariation in Schieberichtung kann für eine Längenwachstumskorrektur bei einem Kind und/oder für eine individuelle Valgus- oder Varuseinstellung genützt werden. Sowohl bei der Längenwachstumskorrektur als auch bei der Valgus- oder Varuseinstellung handelt es sich um zeitlich fortschreitende Nachstellungen, d. h. die Orthese kann der körperlichen Entwicklung des Kindes nachgeführt werden. Da die Orthese dem Längenwachstum eines Kindes angepasst werden kann, müssen die Fassungsteile für Unterschenkel und Fuß Spielraum für ein dem Längenwachstum entsprechendes Breitenwachstum aufweisen.

EP 1 086 671 A2 zeigt eine Orthese mit einem Oberschenkel-Fassungsteil und einem Unterschenkel-Fassungsteil. Die beiden Teile sind über Stangen und über ein auf Kniehöhe verschwenkbares Gelenk miteinander verbunden, sodass eine Kniebeugebewegung möglich ist. Der Längsabstand zwischen dem sich auf Kniehöhe befindenden Gelenk und dem Unterschenkelteil bzw. auch dem Oberschenkelteil wird zur Anpassung an die jeweiligen Körperproportiönen durch jeweils eine Längseinstelleinrichtung eingestellt. Die Stangen können mehr oder weniger tief in ihre jeweilige Einstelleinrichtung eingeführt werden. Dabei werden die Stangen in ihrer jeweiligen Einstelleinrichtung bei dem gewünschten Abstand zwischen Gelenk und Fassungsteil verriegelt.

US-A-2934064 zeigt eine Stützstruktur für ein Bein für verschiedene Gebrechen desselben, insbesondere für das Knie- und Sprunggelenk. Es ist gezeigt, die Stützstruktur mit Hilfe einer Manschette unterhalb oder oberhalb des Knies zu befestigen. Seitlich des Unterschenkels verlaufen Streben. Diese Streben sind gelenkig mit einem Fußteil verbunden. Das Fußteil ist in etwa U-förmig ausgebildet und zum Einbringen in einen Schuh ausgelegt. Der Fuß tritt beim Anziehen des Schuhs zwischen die Schenkel des Fußteils und auf eine untere Fußplatte. In den Längsstreben ist eine Längeneinstelleinrichtung vorgesehen. Die Längeneinstelleinrichtung weist eine Druckfeder auf, die auf ein Klemmelement mit Handgriff einwirkt. Die Druckfeder spannt das Klemmelement in eine Verriegelungsstellung vor, die die Längeneinstelleinrichtung und damit die eingestellte Orthesenlänge verriegelt. Durch Betätigung des Handgriffs wird die Verriegelung gelöst und kann die Orthesenlänge verstellt werden. Insgesamt ist die Stützung dazu ausgelegt, einen moderaten Zug auf das Bein vom Fuß bis zur obersten Befestigung auszuüben. Dabei ist die vorbekannte Orthese auf Grund der oben beschriebenen Konstruktion jedoch nur in der Lage, einen relativ geringen, vorher auf das Bein aufgebrachten Zug durch Einstellung einer geeigneten Orthesen(über)länge zu halten. Für das vorherige Aufbringen des Zuges auf das Bein ist eine gesonderte Vorrichtung erforderlich.

WO 91/13604 A zeigt eine Stütz- und Haltevorrichtung für ein Sprunggelenk, mit der das Gelenk programmierbar zyklisch bewegt werden kann, wobei der physiologische Bewegungsablauf erhalten bleibt. Die Vorrichtung hat eine obere Manschette zur Befestigung am Unterschenkel. Ein Fußfassungsteil in Form einer Fersenkappe wird mit Hilfe eines Klettverschlusses am Fuß befestigt. Fußkappe und Manschette sind über ein Gestänge miteinander verbunden, wobei sowohl die Manschette als auch das Fußteil jeweils über Kugelgelenke mit dem Gestänge verbunden sind. Das Gestänge weist zwischen der Manschette und der Fersenkappe eine zylindrische Längeneinstelleinrichtung auf. Es wird insbesondere gezeigt, Federn in solche Einstelleinrichtungen aufzunehmen, um die Befestigungen auseinander zu drücken. Insgesamt sollen diese Vorrichtungen dazu ausgelegt sein, eine bestimmte Kraft graduell und kontinuierlich oder diskret und zyklisch auszuüben. Dabei soll das von der Vorrichtung eingefasste Gelenk je nach Wunsch gebeugt oder gestreckt werden können. Die Ferse wird heruntergedrückt oder hochgezogen; auf Grund der eben erwähnten Kugelgelenke bewegt sich dabei das Sprunggelenk physiologisch. Unter "Strecken eines Gelenks" wird in dieser Druckschrift die Vergrößerung des Winkels verstanden, den die beiden an das Gelenk anschließenden Körperteile miteinander einschließen, nicht jedoch das Auseinanderziehen eines Gelenkspalts. Die Längeneinstelleinrichtung weist zwei Verstellmöglichkeiten auf. Zum einen kann mittels eines Einstellrades die elastische Verformung der Feder und damit die Federkraft eingestellt werden, mit die Ferse (zur Mobilisierung des Gelenks) nach unten gedrückt wird. Zum anderen kann durch Betätigung eines weiteren Rades die Gesamtlänge zwischen den Kugelgelenken zur Anpassung an die unterschiedlichen Körpergrößen der Patienten eingestellt werden.

US-A-4099525 zeigt eine Stützvorrichtung für Pferde mit einem starren länglichen Unterstützungsteil, einem Hufteil und einem Beinteil. Mit der Stützvorrichtung kann der Winkel zwischen Huf und Bein eingestellt werden, insbesondere der vordere Teil des Hufes angehoben und damit der hintere Teil des Hufes abgesenkt werden, um verkürzte Sehnen wieder zu längen. Hufteil und Beinteil sind über das längliche Unterstützungsteil miteinander verbunden. Das Hufteil ist flächig ausgestaltet und unterhalb des Hufs befestigt. Das Unterstützungsteil ist als Gewindestange ausgebildet und verläuft längs der Vorderseite des Beins. Das Beinteil ist über eine Öse mit einer darüber und eine darunter angeordneten Mutter an dem länglichen Unterstützungsteil befestigt. Über das Gewinde kann der Abstand zwischen dem oberen Beinteil und dem Hufteil eingestellt werden. Der vordere Teil des Hufs wird so angehoben und der hintere Teil des Hufs abgesenkt.

US 2005/0273028 A1 zeigt eine Orthese zur Umfassung des unteren Teils des Unterschenkels sowie des Sprunggelenks und des Fußes. Die Orthese ist in Segmente aufgeteilt, um die durch die Orthese ausgeübten Reaktionskräfte den natürlichen Reaktionskräften nachzubilden.

WO 97/13487 A zeigt eine Unterstützung mit einem Fußteil und einem Wadenteil, welche schwenkbar unterhalb des Knöchels miteinander verbunden sind. Die beiden Teile sind auch über eine Kompressionsfeder miteinander verbunden, um die Schwenkbewegung elastisch zu behindern.

Der Erfindung liegt die Aufgabe zugrunde, eine Lehre anzugeben, mit der flexible knöcherne Strukturen besonders schonend auseinandergezogen und gestreckt gehalten werden können.

Diese Aufgabe wird durch die Merkmale des Anspruchs 1 gelöst.

Bevorzugte Ausgestaltungen der Erfindung sind in abhängigen Ansprüchen angegeben und werden im Folgenden näher erläutert.

Die Erfindung beruht auf der Beobachtung, dass beliebige flexible knöcherne Strukturen - also nicht knöchern miteinander verwachsene Strukturen - mit einer Orthese gut fixiert werden können; auch gegen den Widerstand von Bändern und Muskeln. Weiter beruht die Erfindung auf der Idee, eine Orthese für einen gestreckten Halt auf den zwei gegenüberliegenden Seiten einer flexiblen knöchernen Struktur mit je einer Befestigungseinrichtung, also mehrteilig, auszulegen sowie die Befestigungseinrichtungen über eine Einstelleinrichtung zum Herstellen unterschiedlicher Abstände zwischen den Befestigungseinrichtungen miteinander zu verbinden.

Vor allem verlangt die Orthese nicht notwendig einen Eingriff in das Gewebe. Sie kann ggf. einfach äußerlich angelegt werden. In der Regel wird es besonders einfach sein, die Orthese bei verkürztem Abstand zwischen den Befestigungseinrichtungen anzulegen und nach dem Anlegen über die Einstelleinrichtung, soweit gewünscht, den verlängerten Abstand herzustellen. Dabei wird der Abstand zwischen den Befestigungseinrichtungen über die Einstelleinrichtung hergestellt. Das heißt, die Einstelleinrichtung wird durch eine Bedienperson betätigt, woraufhin die Einstelleinrichtung wahlweise einen verkürzten oder einen verlängerten Abstand herstellt. Die Einstelleinrichtung kann insbesondere so ausgelegt sein, dass eine Bedienperson lediglich ein Bedienelement zu betätigen hat, etwa einen Knopf oder einen Hebel, woraufhin die Einstelleinrichtung den gewählten Abstand herstellt, etwa die Befestigungseinrichtungen mit einer gewissen Kraft auseinander drückt. Insbesondere wird es einer Bedienperson nicht abverlangt, selber die Befestigungseinrichtungen auseinander zu drücken. Dies geschieht über die Einstelleinrichtung.

Die anatomisch formschlüssigen Schalenteile dienen zum Abstützen der Orthese an knöchernen Elementen bzw. skelettalen Abstützflächen, d. h. an prominenten knöchernen Strukturen. Die Schalenteile umfassen den Körper auf jeweils einer Seite der knöchernen Struktur idealerweise vollständig. Mit Hilfe der Schalenteile können bestimmte Strukturen gezielt entlastet bzw. gestreckt oder gedehnt werden. Es kann also ggf. ein bestimmter intraartikulärer Spalt mit Hilfe der Erfindung behandelt und ein ggf. benachbarter intraartikulärer Spalt geschont werden.

Die Schalenteile können etwa aus kohlefaserverstärktem Gießharz bzw. einem Gießharzlaminat hergestellt werden. Für die Innenhüllen hat sich Polyethylenschaum bewährt.

Wenn die Befestigungseinrichtungen besonders gut fixiert werden sollen und/oder die Befestigungseinrichtungen mit einer vergleichsweise großen Kraft auseinandergedrückt werden sollen, kann es bevorzugt sein, die Erfindung mit den Pins eines "*Fixateur externe"* zu kombinieren. Beispielsweise können die Pins durch die Befestigungseinrichtungen, insbesondere entsprechende Schalenteile, hindurchgetrieben werden, um in einer knöchernen Struktur verankert zu werden.

Die flexible knöcherne Struktur kann mit der Orthese im gestreckten Zustand ruhig gehalten werden. Durch einen hinreichend verlängerten Abstand zwischen den Befestigungseinrichtungen kann die flexible knöcherne Struktur zwischen den Befestigungseinrichtungen auch auseinander gezogen werden; nicht knöchern miteinander verwachsene Strukturen können aufgedehnt werden.

Das Auseinanderziehen flexibler knöcherner Strukturen kann diese vorteilhaft entlasten bzw. die korrekten anatomischen Verhältnisse können ggf. so hergestellt werden. Je nach Einsatzzweck, kann auch eine Teilentlastung schon vorteilhaft sein. Je nach Ausgestaltung der erfindungsgemäßen Orthese werden die knöchernen Bestandteile nicht nur auseinandergezogen, sondern können auch in lateraler Richtung relativ zueinander versetzt werden. Beispielsweise kann es die Erfindung erlauben, bei der sogenannten "Schublade" im Kniebereich nicht nur Oberschenkel und Unterschenkel auseinander zu ziehen, sondern auch den Unterschenkelknochen in eine anatomisch korrekte Position zu versetzen.

Grundsätzlich kann die Orthese vorteilhaft am menschlichen oder auch am tierischen Körper eingesetzt werden.

Flexible knöcherne Strukturen im Sinne der Erfindung sind etwa beliebige Gelenke mit intraartikulärem Spalt oder Pseudarthrosen, korrespondierende von einem Zwischenraum abgehende Gelenkteile oder Frakturen. Grundsätzlich kann jede nicht knöchern verwachsene Struktur durch die Orthese gedehnt bzw. auseinandergezogen werden.

Beispielsweise kann die Orthese bei folgenden Indikationen entweder zur Reposition, Distraktion oder Mobilisation vorteilhaft eingesetzt werden:

Betreffend das obere Sprunggelenk, das untere Sprunggelenk und das Knie etwa bei Osteachondrosis dissecans (konservativ und postoperativ), postoperativ nach Knorpeltransplantation, zur Interimsbehandlung bei Endoprothesenwechsel (septisch) und bei Endprothesen mit postoperativen Mobilisierungsproblemen.

Betreffend den Ellenbogen bei posttraumatischen Kontrakturen und bei Endprothesen mit postoperativen Mobilisierungsproblemen.

Grundsätzlich kann man im Fall der Algodystrophie (nach M. Sudeck) zur schonenden Anwendung einer Mobilisierung die Orthese einsetzen.

Die Orthese kann beispielsweise an den Extremitäten aber auch am Rumpf eingesetzt werden, z.B. nach Wirbelkörperfrakturen oder allgemein in Gelenk- oder wirbelsäulenentlastender Weise. Bei entsprechend filigraner Konstruktion ist die Orthese auch zum Einsatz an den Füßen, Händen, Zehen oder Fingern geeignet.

Allgemein kann die Orthese bei Arthrose, postoperativer Nachbehandlung, Ausheilung oder auch ansonsten bei Entzündungen und/oder Schmerzen sinnvoll einsetzbar sein bzw. auch zur gezielten Knochenverlängerung oder Achsenkorrektur.

Im Falle einer Extremität umgreifen die Befestigungseinrichtungen diese hinreichend fest proximal und distal der betreffenden knöchernen Struktur.

Vorzugsweise kann über die Einstelleinrichtung nicht nur ein verkürzter und ein verlängerter Abstand, sondern mehrere weitere Abstände, idealerweise sogar ein Kontinuum von Abständen, zwischen den Befestigungseinrichtungen eingestellt werden.

Vorzugsweise ist die Orthese dazu ausgelegt, frei wählbare Abstände zwischen der ersten und der zweiten Befestigungseinrichtung herzustellen, soweit diese in ein Intervall von einem minimalen Abstand bis zu einem maximalen Abstand fallen.

Diese Auslegung ermöglicht es etwa, den Abstand zwischen den Befestigungseinrichtungen dem Fortschritt einer Behandlung entsprechend nachzuführen.

Bei einer bevorzugten Ausführungsform ist eine Schiene in Streckrichtung unbeweglich, idealerweise starr, mit der ersten Befestigungseinrichtung verbunden. Die zweite Befestigungseinrichtung kann in dieser Schiene geführt werden. Es können etwa zwei als Schienen fungierende metallische Streben gegenüberliegend seitlich an der ersten Befestigungseinrichtung befestigt und in Richtung zweite Befestigungseinrichtung ausgerichtet sein. Zur Führung der zweiten Befestigungseinrichtung in den Schienen können diese jeweils eine länglichen Spalt aufweisen, in welche die zweite Befestigungseinrichtung etwa über Führungsstifte eingreift. Grundsätzlich kann auch eine einzige Schiene zur Führung ausreichend sein. Je nach Anforderung kann die Schiene beispielsweise Richtung Körpermitte oder Körperaußenseite gelegen sein. Wenn gewünscht, kann mit Hilfe von Schienen die Bewegung der Befestigungseinrichtungen besonders gut auf die Streckrichtung alleine festgelegt werden; in einer solchen Ausführungsform wäre für die Befestigungseinrichtungen lediglich ein Bewegungsfreiheitsgrad in Streckrichtung gegeben.

Eine Führung der Relativbewegung der Befestigungseinrichtungen in Streckrichtung lässt sich über eine Schiene konstruktiv einfach und stabil realisieren.

Vorzugsweise umfasst die Einstelleinrichtung eine Schaltfedereinrichtung, also eine federbelastete Einrichtung, die zwischen verschiedenen Zuständen hin- und hergeschaltet werden kann. In einem Schaltzustand wird über die Schaltfedereinrichtung der verkürzte Abstand zwischen den Befestigungseinrichtungen eingestellt; in einem anderen Schaltzustand der verlängerte Abstand zwischen diesen.

Bei der Schaltfedereinrichtung kann es sich um eine Art von Totpunktfeder handeln. Die Schaltfedereinrichtung kann etwa einen Arbeitszylinder, insbesondere eine Gasdruckfeder, umfassen.

Eine Verlängerung bzw. eine Verkürzung der Einstelleinrichtung kann bei einem Arbeitszylinder durch Ausfahren bzw. Einziehen der Kolbenstange umgesetzt werden. Wird etwa die erste Befestigungseinrichtung an dem dem Zylindergehäuse abgewandten Ende der Kolbenstange befestigt und die zweite Befestigungseinrichtung an dem Zylindergehäuse, so schlagen sich die Zustandänderungen des Arbeitszylinders in Abstandänderungen zwischen den Befestigungseinrichtungen nieder.

Es ist auch bevorzugt, eine Einstelleinrichtung mit einer Gewindestange einzusetzen. Hier kann beispielsweise die erste Befestigungseinrichtung derart mit der Gewindestange verbunden sein, dass keine Relativbewegung zwischen Gewindestange und der ersten Befestigungseinrichtung in Streckrichtung möglich ist. Die andere Befestigungseinrichtung kann dann zur Einstellung des Abstands, etwa über eine drehbar gehaltene Mutter, an dem Gewinde entlanggeführt werden. Grundsätzlich können auch Zahnstangen oder Schnecken für die Einstelleinrichtung verwendet werden.

Vorzugsweise ist die Einstelleinrichtung dazu ausgelegt, den Abstand zwischen der ersten Befestigungseinrichtung und der zweiten Befestigungseinrichtung starr herzustellen. Einmal eingestellt, soll ein bestimmter Abstand zwischen den Befestigungseinrichtungen bis zur nächsten Einstellungsänderung unveränderlich sein.

Alternativ ist es bevorzugt, die Einstellungseinrichtung so auszulegen, dass der Abstand zwischen der ersten Befestigungseinrichtung und der zweiten Befestigungseinrichtung federnd-nachgiebig hergestellt ist. Hier ist der Abstand zwischen den Befestigungseinrichtungen auch bei einer bestimmten Einstellung der Einstelleinrichtung nicht fix. Wird die Orthese in Streckrichtung zusammengedrückt, so wird der Abstand zwischen den Befestigungseinrichtungen gestaucht. Lässt die Belastung nach, so werden die Befestigungseinrichtungen über eine Federeinrichtung wieder auseinander gedrückt. Als Federeinrichtung kann die Einstelleinrichtung etwa eine Feder oder einen Gasdruckzylinder aufweisen.

Wird der Abstand federnd nachgiebig hergestellt, kann auch die Kraft, mit der die Befestigungseinrichtungen auseinandergedrückt werden, vorgegeben werden.

Eine bevorzugten Ausführungsform ist ausgelegt zum Halten, insb. Auseinanderziehen, des Sprunggelenks. Diese Ausführungsform weist eine Unterschenkelschale zum Fixieren der Orthese an dem Unterschenkel auf. Dabei kann die Unterschenkelschale so ausgelegt sein, dass sie an dem Schienbeinplateau und den Schienbeinkondylen abgestützt werden kann. Weiter weist sie ein Fußteil zum Fixieren der Orthese an dem Fuß, vorzugsweise mit einer zumindest abschnittsweisen ringförmigen Fassung für diesen, auf. Für die Ausführung einer solchen zumindest abschnittsweisen ringförmigen Fassung ist es vorteilhaft, wenn diese im Vergleich zu den zu fixierenden Strukturen inelastisch ist.

Nach jüngsten medizinische Erkenntnissen können sich im Sprunggelenk, insbesondere beim Talus, posttraumatisch oder postoperativ, Verklebungen ergeben. Hier ist es besonders wichtig, die Sprunggelenksstruktur zu entlasten, um die Mobilität zu erhalten. Bisher griff man bei einer Sprunggelenksverletzung etwa auf die sogenannte Allgöwer-Orthese zurück. Hier handelt es sich etwa um eine Unterschenkelorthese, die etwa im Kniebereich abgestützt werden kann und die ohne jede Selektion sämtliche Strukturen unterhalb der Abstützung entlastet.

Bei einer bevorzugten Ausführungsform der Erfindung ist die Orthese zur Fassung einer zu korrigierenden Extremität an auseinander liegenden Stellen ausgelegt. Dabei sind die betreffenden Befestigungseinrichtungen, insbesondere vor dem Anziehvorgang, zumindest teilweise voneinander trennbar und noch nach dem Anziehvorgang in Bezug zueinander justierbar, wobei die gegenseitige Justierung durch eine leicht herstellbare Verbindung in vorgegebener gegenseitiger Position erfolgt. Konstruktive Details lassen sich der DE 101 14 032 B9 entnehmen.

Die Befestigungseinrichtungen lassen sich also auch als Fassungsteile zur Fassung einer zu korrigierenden Extremität an auseinander liegenden Stellen auffassen.

Orthesen zur Korrektur von Gelenkfehlstellungen sind grundsätzlich bekannt. Etwa zur Korrektur von Fehlstellungen im Sprunggelenk- oder Kniebereich eines Beines, aber auch im Handwurzel- und/oder Ellbogenbereich eines Armes. Damit werden gegenüber ihrer gegenseitigen Regellage versetzte Extremitätenabschnitte mittels sanfter Gewalt zumindest in Richtung auf ihre gegenseitige Regellage gedrängt. Bei vielen herkömmlichen Orthesen bereitet das Anziehen unter der betreffenden Krafteinwirkung Schwierigkeiten und bedarf zumindest in den allermeisten Fällen einer Hilfsperson, die zudem mit Sachverstand und Geduld vorzugehen hat. Hier schafft die Orthese nach dieser bevorzugten Ausführungsform Abhilfe.

Dadurch, dass die an den Extremitätenabschnitten angreifenden Fassungsteile, also die Befestigungseinrichtungen, nach dem Anziehvorgang in Bezug zueinander justierbar sind, ist es vergleichsweise leicht und unter Umständen sogar ohne Hilfsperson möglich, die betreffende Orthese anzuziehen, und erst dann die einzelnen Fassungsteile der Orthese in Bezug zueinander in die vorgesehene Korrekturstellung zu bringen. Dazu sind die einzelnen Fassungsteile vorzugsweise vollkommen voneinander trennbar, wobei die gegenseitige Justierung durch eine leicht herstellbare Verbindung in vorgegebener gegenseitiger Position erfolgt. Dies kann ungeachtet einer gewünschten gegenseitigen Beweglichkeit, beispielsweise für das Kniegelenk der Fall sein. Abgesehen von einer Erleichterung des Anziehens zeichnet sich diese Orthese aber vor allem auch noch dadurch aus, dass sie auf eine einfache Weise eine manuelle intraartikuläre Korrektur von Gelenkluxationen, Fehlrotationen, Fehltranslationen, longitudinalen Achsenfehlstellungen und dergleichen ermöglicht.

Beispielsweise können Schalenteile, die auch als Befestigungseinrichtung nach der Erfindung fungieren, über Schienen miteinander verbunden sein. Dabei kann etwa, wie oben dargestellt, eine der Befestigungseinrichtungen fix mit den Schienen verbunden sein und die andere der-Befestigungseinrichtungen in diesen Schienen geführt werden. Die Schienen können etwa nach dem Anziehen über Schraubverbindungen angebracht werden. Gegebenenfalls kann in den Schalenteilen eine metallene Grundplatte zur Stabilisierung und Aufnahme der Schrauben vorgesehen sein.

Der Rückfuß umfasst insbesondere das Sprungbein (Talus) und das Fersenbein (Calcaneus). Projiziert in die Transversalebene liegen physiologische talocalcaneale Winkel im Bereich von etwa 30° bis etwa 35°.

Bei bestimmten Deviationen im Rückfuß liegen unter anderem abweichende talocalcaneale Winkel vor. So ist dieser Winkel bei dem so genannten Klumpfuß kleiner und bei dem Knickfuß größer.

Es ist bekannt, entsprechende Deviationen im Rückfuß operativ oder konservativ zu behandeln.

Zur konservativen Behandlung des Klumpfußes ist der so genannte Klumpfußgips bekannt. Hier wird grundsätzlich ein Oberschenkelgips angelegt, um eine hinreichende Redression des Fußes nach außen zu erzielen. Damit der Fuß mit dem Klumpfußgips als Ganzes gegenüber dem Oberschenkel nach außen redressiert werden kann, muss das Kniegelenk gebeugt sein. Es ist auch bekannt, eine entsprechend geformte und starre Orthese anzugeben.

Weiter ist bei einem solchen Verfahren eine Redression von 70° bis 80° abweichend von der Neutral-Null-Stellung (s.u.) erforderlich, da sich die Drehung über verschiedene Teile des Beins verteilt; dies belastet insbesondere den Bandapparat im gesamten Bein.

Bei der Klumpfußbehandlung nach Ponseti erfolgt eine Korrektur schrittweise über das sukzessive Tragen von mehreren, bspw. drei bis acht, Gipsen für jeweils unterschiedliche Redressionen.

Bevorzugt ist die Orthese auch zur Behandlung pathologischer Deviationen im Rückfuß ausgelegt, mit einer oberen Fixiereinrichtung zum Fixieren der Sprungbeinrolle in der Knöchelgabel und einem Fußteil, das zur Aufnahme der subtalaren Fußplatte eingerichtet ist und relativ zu der oberen Fixiereinrichtung in der Transversalebene zwischen mindestens zwei Ausrichtung drehbar und in jeder der Ausrichtungen lösbar fixierbar ist. Die Fixiereinrichtung kann dabei der ersten Befestigungs- und das Fußteil der zweiten Befestigungsvorrichtung entsprechen; oder umgekehrt.

Die Ausführungsform beruht auf der Erkenntnis, dass zur konservativen Korrektur der Winkelstellung zwischen dem Sprungbein und der darunter liegenden subtalaren Fußplatte, zu welcher das Fersenbein gehört, es vor allem der Fixierung der Sprungbeinrolle, als Teil des Sprungbeins, in ihrer Einbettung an der Knöchelgabel bedarf. Dazu dient die oberen Fixiereinrichtung.

Weiter beruht die Ausführungsform auf der Idee, die subtalare Fußplatte relativ zu dem fixierten Sprungbein in der Transversalebene drehbar und bei hinreichender Redression fixierbar in der Orthese aufzunehmen. Dazu weist die Orthese ein Fußteil auf, welches zur Aufnahme zumindest der subtalaren Fußplatte, oder auch des ganzen Fußes, eingerichtet ist und welches relativ zu der Fixiereinrichtung der Orthese in der Transversalebene drehbar und in verschiedenen Ausrichtungen, zumindest in einer Fehlstellung und in einer Korrekturstellung, in der Transversalebene lösbar fixierbar ist.

Wird auf eine Drehung des Fußteils in der Transversalebene abgestellt, so heißt dies nicht, dass eine entsprechende Orthese auf eine Bewegung in der Transversalebene beschränkt ist und keine weiteren Komponenten aufweisen darf. Vielmehr wird unter einer Drehung in der Transversalebene jede Bewegung verstanden, bei der die Projektion in die Transversalebene im Ergebnis an einer Drehung teilnimmt. Sinngemäß gilt das gleiche auch für weiter unten erwähnte Drehungen des Fußteils in weiteren Ebenen. So kann bei einer bevorzugten Ausführungsform (s.u.) bspw. mit einer Drehung des Vorfußes nach oben auch eine Spitzfußkomponente behandelt werden.

Die Ausrichtung des Fußteils kann relativ zu der so genannten Neutral-Null-Stellung angegeben werden. Diese Stellung nimmt der Fuß im Normalfall beim aufrechten Stand des Menschen ein.

Zur Korrektur bspw. eines Klumpfußes kann der Fuß in Fehlstellung in dem Fußteil aufgenommen und die Sprungbeinrolle in der Knöchelgabel über die Fixiereinrichtung fixiert werden. Anschließend kann das Fußteil in der Transversalebene nach außen gedreht und dort fixiert werden.

Zur Korrektur eines Knickfußes wird das Fußteil nach innen gedreht. Angestrebt wird letztlich ein physiologischer Winkel zwischen dem Fersenbein und dem Sprungbein. Zur Behandlung bietet sich jedoch, zumindest zunächst, eine moderate Überkorrektur an. Bei hinreichender Redression wird dabei das Fußteil fixiert; diese Ausrichtung des Fußteils wird solange beibehalten, wie es die Behandlung erfordert.

Vorzugsweise ist das Fußteil jedoch zwischen mehr als zwei Ausrichtungen in der Transversalebene drehbar und in jeder dieser Ausrichtungen lösbar fixierbar, idealerweise ist die Ausrichtung des Fußteils in der Transversalebene sogar kontinuierlich einstellbar und dabei in jeder seiner Ausrichtungen lösbar fixierbar. Die Drehung des Fußteils kann etwa durch manuelle Führung an dem distalen Ende des Fußes erfolgen.

Insgesamt ermöglicht es diese Ausführungsform auch pathologische Deviationen im Rückfuß besonders gezielt und einfach zu behandeln. Strukturen, die nicht in unmittelbarer Nähe der zu korrigierenden Strukturen liegen, werden geschont. Zumindest bei einem günstigen Behandlungsverlauf kann auf einen Gips verzichtet werden.

Weiter ist es möglich, die Redression des Fußes im Laufe der Behandlung an den Fortschritt derselben anzupassen. Insbesondere erübrigt sich hier ggf. das Anlegen mehrerer Gipse nacheinander.

Außerdem ermöglicht die Orthese ein Anlegen in der Fehlstellung, was die Handhabbarkeit steigert.

Besonders vorteilhaft ist die Möglichkeit, bei der Behandlung einer pathologischen Deviation im Rückfuß flexible knöcherne Strukturen gestreckt halten beziehungsweise auseinander ziehen zu können. Beispielsweise kann bei einem postoperativen Klumpfuß der Abstand zwischen Fußteil und oberen Fixiereinrichtung an eine gegebenenfalls vorhandene Schwellung angepasst werden. Ist etwa eine Arthrose in Verbindung mit einer Deviationen im Rückfuß gegeben, kann neben der Behandlung der Korrektur eine dauerhafte Dehnung in Streckrichtung sinnvoll sein.

Bei einer bevorzugten Ausführungsform der Erfindung ist das Fußteil in der Transversalebene zweidimensional, also insb. nach vorn und hinten und nach links und nach rechts, verschiebbar und in jeder dieser Positionen lösbar fixierbar. Auf diese Weise ist es möglich, bei beliebiger Drehung des Fußteils, die Orthese so einzustellen, dass der biomechanische Drehpunkt im Sinus Tarsi - in der Mitte zwischen der Furche zwischen dem Sprung- und Fersenbein - liegt, seiner physiologischen Lage entsprechend.

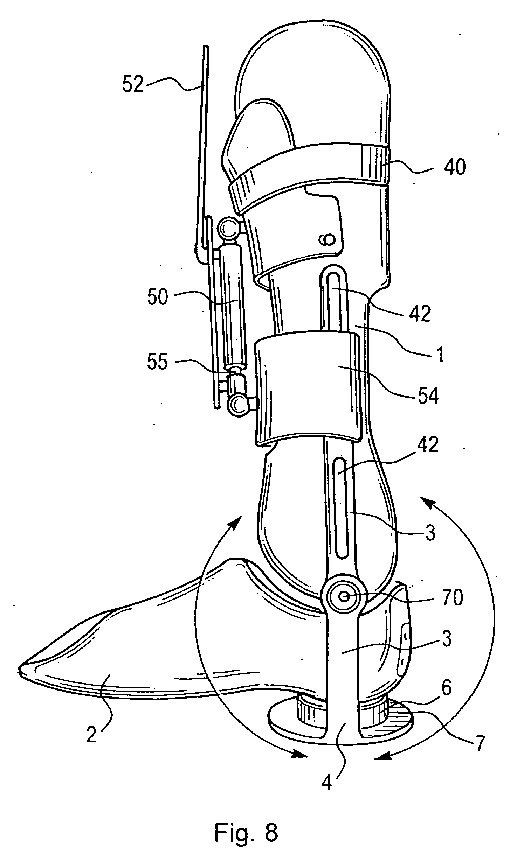

Bei dieser bevorzugten Ausführungsform ist es auch grundsätzlich möglich, neben einem Auseinanderziehen der Befestigungseinrichtungen, deren Position auch quer zur Streckrichtung relativ zueinander einzustellen (vgl. Fig. 8).

Ggf. kann auch schon eine Orthese vorteilhaft eingesetzt werden, bei der das Fußteil lediglich medio-lateral entlang einer Geraden quer zu der medialen Ebene verschiebbar und entlang dieser Geraden lösbar fixierbar ist.

Vergleichbar zu der Sprachregelung bei den Drehungen, wird unter einer Verschiebung in der Transversalebene jede Bewegung verstanden, deren Projektion in die Transversalebene an einer Verschiebung in derselben teilnimmt.

Um die Orthese noch flexibler einsetzen zu können, ist es bevorzugt, sie so auszulegen, dass das Fußteil auch in der Sagittal- und/oder Frontalebene drehbar und lösbar fixierbar ist, also Drehbarkeit in jede Raumrichtung, etwa den anatomischen Achsen folgend, gegeben ist. Die entsprechenden Drehungen können auch aus anderen Bewegungen zusammengesetzt sein, etwa aus einer Kipp- und einer Translationsbewegung. So kann der Fuß in die therapeutisch erforderliche Position gedreht werden, ohne beispielsweise das Sprungbein in eine Fehlposition zu drängen. Idealerweise ist das Fußteil auch bezüglich dieser Drehungen für ein Kontinuum von Ausrichtungen lösbar fixierbar. Es sind so beliebige Pronationen und Supinationen einstellbar.

Es sollten jedoch, vor allem in der Sagittalebene, keine zu großen Abweichungen von der Neutral-Null-Stellung eingestellt werden, da ansonsten das Risiko für eine Luxation zu hoch ist. So sollte etwa der Winkel zwischen dem Fuß und dem Unterschenkel, bei gleichzeitiger Drehung in der Transversalebene, 90° nicht wesentlich überschreiten, da ansonsten die Sprungbeinrolle aus der Knöchelgabel springen kann. Anders ausgedrückt, vorzugsweise beträgt der Winkel zwischen Fuß und Unterschenkel im redressierten Zustand höchstens 120°; besser höchstens 100°.

Vorzugsweise weist die oberen Fixiereinrichtung ein Schalenteil, oder auch mehrere Schalenteile, für den Unterschenkel auf, welches den Innen- und den Außenknöchel zur mechanischen

Sicherung der Sprungbeinrolle in physiologischer Position distal umklammert. Über die Umklammerung wird genügend Druck für eine hinreichende Fixierung ausgeübt. Dazu kann das Schalenteil etwa mit Riemen, ggf. mit Klettverschluss, zusammengeschnürt werden. Zur Schonung der Knöchel, können diese etwa mit einem ringförmigen Polymergelkissen geschützt werden und/oder aber auch mit einer tubusförmigen Innenhülle. Auch ansonsten können das oder die Schalenteile zur Verbesserung des Komforts mit Innenhüllen versehen sein. Innenhüllen werden in der Orthopädietechnik auch "Liner" genannt.

Die Schalenteile können etwa aus kohlefaserverstärktem Gießharz bzw. einem Gießharzlaminat hergestellt werden. Für die Innenhüllen hat sich Polyethylenschaum bewährt.

Vorzugsweise weist das Fußteil ein Schalenteil zum Halten des Fußes, bzw. zumindest der subtalaren Fußplatte, auf. Einen sicheren Halt gewährt etwa ein Fußteil, welches den Fuß, bspw. im Bereich der Mittelfußknochen, ringförmig umschließt. Dieses Schalenteil kann auch mit einer Innenhülle ausgestattet sein. Für einen leichten Einstieg in die Orthese kann das Fußteil fersenseitig mit einer abnehmbaren Klappe versehen sein.

Bei einer bevorzugten Ausführungsform ist das Fußteil an einer Halterung befestigt; die ihrerseits an der oberen Fixiereinrichtung befestigt ist. Dabei ist die Halterung als U-förmiger Auftrittsbügel ausgebildet, dessen beide Schenkel an der oberen Fixiereinrichtung befestigt sind und dessen geschlossenes Ende sich unter dem Fußteil befindet, wobei das Fußteil an dem Auftrittsbügel mittels eines Gelenks drehbar gestützt ist.

Die Halterung kann etwa durch zwei seitlich verlaufende an der oberen Fixiereinrichtung starr befestigte Schienen verwirklicht sein. Im unteren Bereich können die Schienen miteinander verbunden sein und den Auftrittsbügel bspw. einstückig ausbilden. Der Auftrittsbügel ist bei gegebener Einstellung des Abstandes zwischen den Befestigungseinrichtungen vorzugsweise fix relativ zu der oberen Fixiereinrichtung.

Das Fußteil kann auch an anderen Teilen der Orthese mittels eines Gelenks drehbar gestützt sein. Auch ein Auftrittsbügel muss nicht unbedingt gegeben sein.

Vorzugsweise handelt es sich bei dem Gelenk um ein Kugelgelenk.

Ist ein Auftrittsbügel gegeben, ist es bevorzugt, das Gelenk in der Transversalebene verschiebbar sowie lösbar fixierbar an dem Auftrittsbügel zu befestigen.

Im Folgenden soll die Erfindung auch anhand von Ausführungsbeispielen näher erläutert werden, ohne dabei die Erfindung durch die Beispiele einschränken zu wollen:
Figur 1 zeigt eine erste erfindungsgemäße Orthese; angelegt an einen Unterschenkel.
Figur 2 sagt eine zweite erfindungsgemäße Orthese.
Figur 3 zeigt nebeneinander Teile einer Orthese zur Behandlung von Deviationen im Rückfuß.
Figur 4 zeigt eine Vorderansicht der Orthese aus Figur 3 in zusammengebautem Zustand.
Figur 5 zeigt eine Rückansicht der Orthese aus Figur 4.
Figur 6 zeigt eine Seitenansicht der Orthese aus den Figuren 4 und 5.
Figur 7 zeigt in einer Rückansicht den unteren Abschnitt einer weiteren Orthese zur Behandlung von Deviationen im Rückfuß
Figur 8 zeigt eine Seitenansicht einer Orthese zur Behandlung pathologischer Deviationen im Rückfuß und zum Auseinanderziehen knöcherner Strukturen.

Für gleiche bzw. einander entsprechende Merkmale werden figurenübergreifend die gleichen Bezugszeichen verwendet.

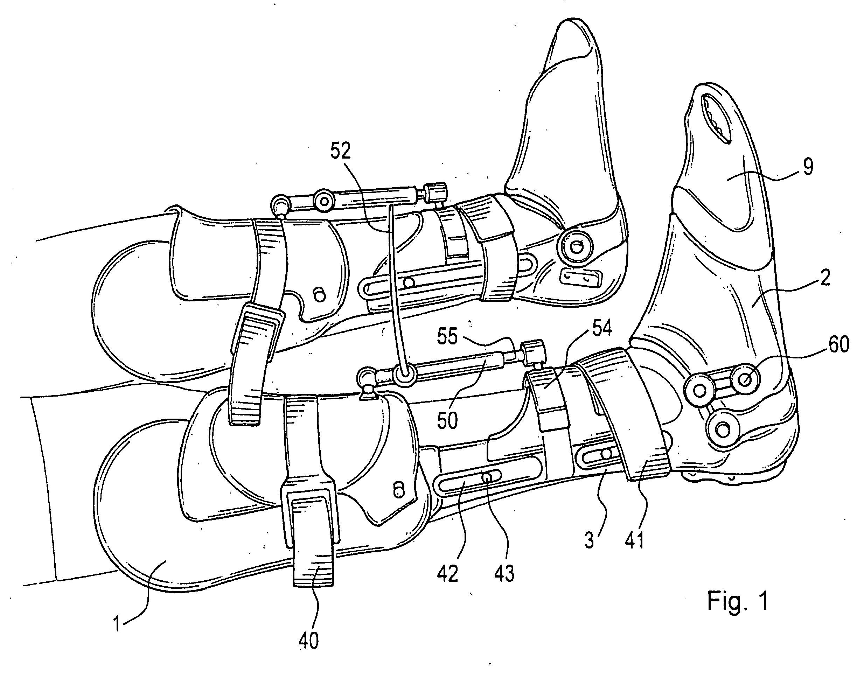

Figur 1 zeigt im Vordergrund eine an einen Unterschenkel angelegte erste erfindungsgemäße Orthese. Eine anatomisch formschlüssige mehrteilige Unterschenkelschale 1 umgibt den Unterschenkel kurz unterhalb des Knies. Die äußere Hülle der Unterschenkelschale 1 besteht aus einem Gießharzlaminat; sie ist ausgefüttert mit einer Innenhülle aus Polyethylen. Die Unterschenkelschale 1 ist an dem Schienbeinplateau und den Schienbeinkondylen abgestützt, um einen sicheren Halt zu gewähren. Mit einem die Unterschenkelschale 1 umgebenden Riemen 40 kann diese zur Verbesserung des Halts an den Unterschenkel gedrückt werden.

Der Fuß wird von einer anatomisch formschlüssigen Fußschale 2, bestehend aus dem gleichen Gießharzlaminat wie die Unterschenkelschale 1, gehalten, welche den Fuß im Bereich der Mittelfußknochen ringförmig umfasst. Eine Innenhülle 9 aus Polyethylen zur Einbettung des Fußes ist hier gut zu erkennen.

Im Bereich der Ferse bzw. Knöchel erkennt man eine außen liegende Fußschalenaufhängung 60, welche ein Verkippen der Fußschale 2 relativ zur Transversalebene erlaubt.

Seitliche Schienen 3 führen ausgehend von der Fußschale 2 in Richtung Knie (man erkennt nur die rechte Schiene 3 im Vordergrund; die linke Schiene ist verdeckt durch den Unterschenkel). Die Schienen 3 sind über die Fußschalenaufhängung 60 derart an der Fußschale 2 befestigt, dass sie in Streckrichtung unbeweglich sind.

Die seitlichen Schienen 3 enden etwa mittig zwischen dem Fuß und dem Knie. In diesem Bereich der Schienen 3 befindet sich auf jeder Seite ein etwa 5 cm langer Führungsspalt 42. Die mehrteilige Unterschenkelschale 1 greift auf jeder Seite über einen Führungsstift 43 in den entsprechenden Führungsspalt 42 ein. Werden Fußschale 2 und Unterschenkelschale 1 in Streckrichtung gegeneinander verschoben, so wird die Bewegung durch die Schienen 3 und die in die entsprechenden Führungsspalte 42 eingreifenden Führungsstifte 43 geführt.

Die beiden Schienen 3 sind etwa mittig durch einen um das Schienbein herumgreifenden metallischen Bügel 54 miteinander verbunden. Ein pneumatischer Arbeitszylinder 50 ist im wesentlichen parallel zu der Streckrichtung ausgerichtet und einerseits an dem Bügel 54 befestigt und andererseits weiter oben an der Unterschenkelschale 1. Durch Umlegen eines Hebels 52 an dem pneumatischen Arbeitszylinder 50 kann die Kolbenstange 55 ausgefahren werden. Der Hebel 52 ist in zwei Einstellungen arretierbar. Bei einer der Einstellungen ist die Kolbenstange 55 maximal ausgefahren; bei der anderen Einstellung ist die Kolbenstange 55 weitgehend eingezogen. Entsprechend werden bei maximal ausgefahrener Kolbenstange 55 die Fußschale 2, vermittelt über den Bügel 54 und die Schienen 3, und die Unterschenkelschale 1 auseinander gedrückt. Bei der anderen Einstellung des Hebels 52 werden die Fußschale 2 und die Unterschenkelschale 1 wieder zusammengezogen.

Anstatt mit einem Arbeitszylinder 50 kann der Abstand zwischen der Fußschale 2 und der Unterschenkelschale 1 auch über eine Gewindestange, Zahnstange oder über ein Schneckengetriebe (jeweils nicht gezeigt) eingestellt werden. Mit entsprechenden Ausführungsformen kann der Abstand zwischen der Fußschale 2 und der Unterschenkelschale 1 auch frei wählbar eingestellt werden, soweit ein minimaler Abstand nicht unter- bzw. ein maximaler Abstand nicht überschritten wird.

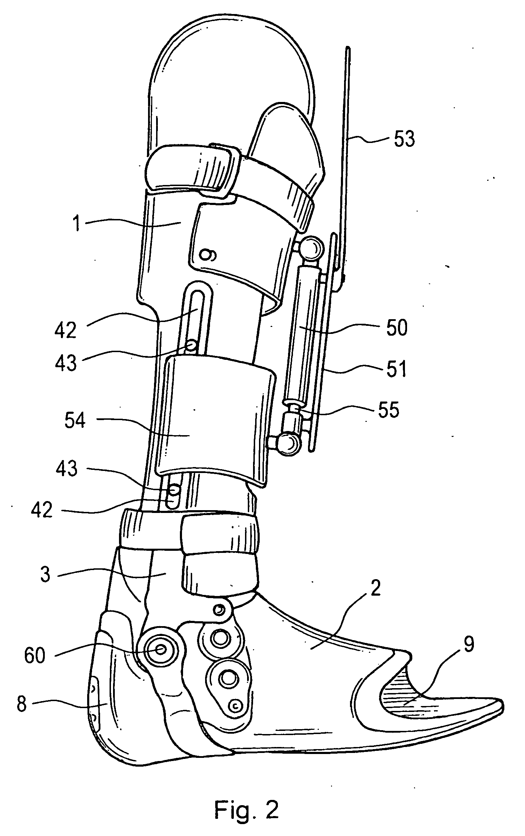

Der Abstand zwischen der Fußschale 2 und der Unterschenkelschale 1 wird über den Arbeitszylinder 50 federnd-nachgiebig eingestellt. Wird die Orthese bspw. in Streckrichtung zusammengerückt, so wird sie aufgrund der federnd-nachgiebigen Abstandseinstellung etwas gestaucht. Lässt die Belastung nach, wird wieder der eingestellte Abstand angenommen. Über den Arbeitszylinder 50, etwa einen hydropneumatischen Zylinder, kann eine Kraft vorgegeben werden, mit der die Befestigungseinrichtungen auseinandergedrückt werden können, beispielsweise 200 bis 600 N. Kleinere und größere Werte sind grundsätzlich auch denkbar. Figur 2 zeigt eine weitere erfindungsgemäße Orthese. Auch diese weist, der Orthese aus Figur 1 vergleichbar, eine entsprechende Unterschenkelschale 1, Fußschale 2 und an der Fußschale 2 befestigte Schienen 3 auf.

Im Unterschied zu der Orthese aus Figur 1 weisen die Schienen 3 hier zwei Führungsspalte 42 auf, in die die Unterschenkelschale 1 über jeweils einen Führungsstift 43 eingreift.

Auch hier wird ein um das Schienbein vorn herumgreifender Bügel 54 verwendet. Hier ist er allerdings aus einem Gießharzlaminat hergestellt, außerdem verdeckt er die Schienen 3 jeweils zwischen den beiden Führungsspalten 42.

Im hinteren Teil der Fußschale 2 erkennt man eine Fersenklappe 8, die für einen leichten Ein- und Ausstieg entfernt werden kann.

Der Abstand zwischen der Unterschenkelschale 1 und der Fußschale 2 wird auch hier über einen pneumatischen Arbeitszylinder 50 eingestellt. Über einen Hebel 53 wird auch hier eine Kolbenstange 55 aus dem Arbeitszylinder heraus gedrückt oder in diesen eingezogen. Der Hebel 53 ist exzentrisch an einem Arretierblech 51 gelagert und wird zur Seite hin betätigt. Sowohl bei ausgefahrener als auch bei eingezogener Kolbenstange wird der Hebel 53 mittels Aussparungen (nicht erkennbar) an dem Arretierblech 51 fixiert.

Bei fixiertem Hebel 53 ist der Abstand zwischen der Fußschale 2 und der Unterschenkelschale 1 unveränderlich eingestellt.

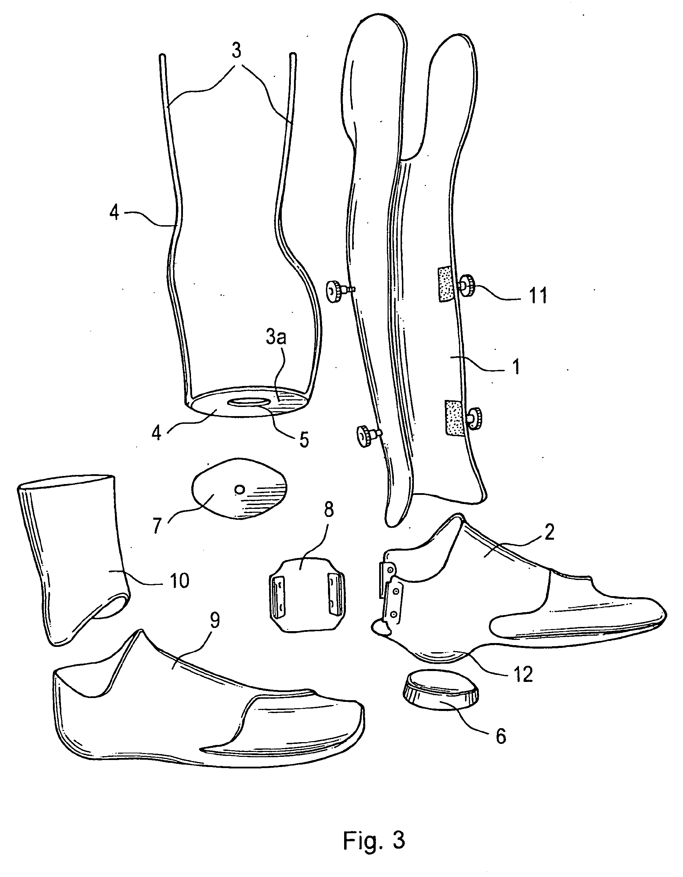

Figur 3 zeigt nebeneinander Teile einer auseinander genommenen Orthese zur Behandlung von Deviationen im Rückfuß. Rechts oben ist eine Unterschenkelschale 1 aus Gießharzlaminat zur Aufnahme eines Unterschenkels gezeigt. Die Unterschenkelschale 1 ist dazu ausgelegt, die Knöchel zu umklammern und so die Sprungbeinrolle in der Knöchelgabel zu fixieren. Die Knöchel können dabei durch eine tubusförmige Innenhülle 10 aus Polyethylen geschützt werden; ergänzend oder alternativ auch mit einem ringförmigen Polymergelkissen (nicht gezeigt).

Der Fuß wird mit einer Fußschale 2 gehalten, welche den Fuß im Bereich der Mittelfußknochen vollständig umschließt. Die Fußschale 2 ist dabei ebenfalls mit einer Innenhülle 9 aus Polyethylen ausgekleidet. Um den Einstieg in die Fußschale 2 zu erleichtern, ist das fersenseitige Ende der Fußschale 2 offen. Es wird mit einer zu verschraubenden Abdeckung 8 nach dem Einstieg verschlossen.

Schienen 3 sind über ein Übergangsstück 3a zum Auftrittsbügel 4 miteinander verbunden, so dass insgesamt eine etwa U-förmige Gestalt ausgebildet wird. Das Übergangsstück 3a weist eine Aussparung 5 auf, um eine verschiebliche sowie lösbare und fixierbare Lagerung der Fußschale 2 zu ermöglichen. Die Unterschenkelschale 1 wird über die Verschraubung 11 zwischen den seitlichen Schienen 3 befestigt.

Eine Pfanne 6 mit einer flachen Seite und einer konvexen Seite wird mit ihrer flachen Seite auf das Übergangsstück 3a aufgelegt. Befestigt wird die Pfanne 6 auf dem Übergangsstück 3a mit einer Halteplatte 7 unterhalb des Auftrittsbügels, wobei die Halteplatte 7 und die Pfanne 6 miteinander verschraubt werden und die entsprechende Schraube (nicht gezeigt) durch die Aussparung 5 hindurchgreift. Bei gelockerter Schraube ist die Pfanne 6 frei in der Ebene des Übergangsstückes 3a, also in der Transversalebene, verschiebbar, soweit es die Aussparung 5 erlaubt.

Die Fußschale 2 weist eine konkave Auswölbung 12 auf, welche mit der Pfanne 6 korrespondiert. Wird die Fußschale 2 über die Auswölbung 12 in die Pfanne 6 gelegt, so kann die Fußschale 2 über die Auswölbung 12 entlang der Oberfläche der Pfanne 6 in allen Ebenen gedreht werden; die Auswölbung 12 fungiert sozusagen als Gelenkkopf eines Kugelgelenks. Befestigt wird die Fußschale 2 in der Pfanne 6 durch eine aus der Pfanne 6 senkrecht nach oben weisende Schraube (nicht gezeigt; diese Schraube kann identisch mit der durch die Aussparung 5 geführten Schraube sein).

Die Auswölbung 12 weist sohlenseitig ein Loch (nicht gezeigt) mit einem Durchmesser von wenigen Zentimetern auf. Auf das Loch, im Inneren der Fußschale 2, wird eine verschiebbare Aufnahme (nicht gezeigt) für die Schraube aus der Pfanne 6 aufgelegt. Die Aufnahme kann von der Form her einem Ausschnitt aus einer Kugeloberfläche entsprechen. Wenn die Schraube in die Aufnahme hinein geschraubt und ausreichend angezogen ist, wird die Fußschale 2 in der Pfanne 6 fixiert. Wird die Schraube gelockert, so kann die Fußschale 2 über die Auswölbung 12 in der Pfanne 6 in alle Raumrichtungen gedreht werden.

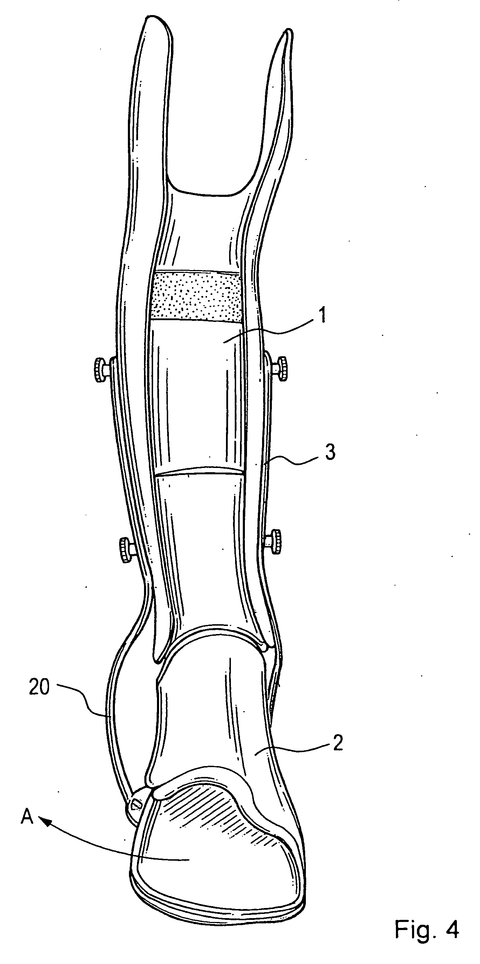

Figur 4 zeigt die Orthese aus den in Figur 3 gezeigten Teilen; zusammengebaut wie dort beschrieben. Es handelt sich um eine Orthese zur Behandlung einer Deviation im Rückfuß mit einer Klumpfußkomponente.

Die Fußschale 2 und die Unterschenkelschale 1 sind in der Neutral-Null-Stellung dargestellt.

Nach dem Anziehen der Orthese wird die Fußschale 2 entlang der Pfeilrichtung A in der Transversalebene nach außen gedreht, um den pathologisch verkleinerten Winkel zwischen dem Fersenbein und dem Sprungbein zu korrigieren. Um eine hinreichende Redression des Fußes erreichen zu können, ist die außen liegende Schiene 3 ausgewölbt 20, um der Drehung A mehr Raum geben zu können.

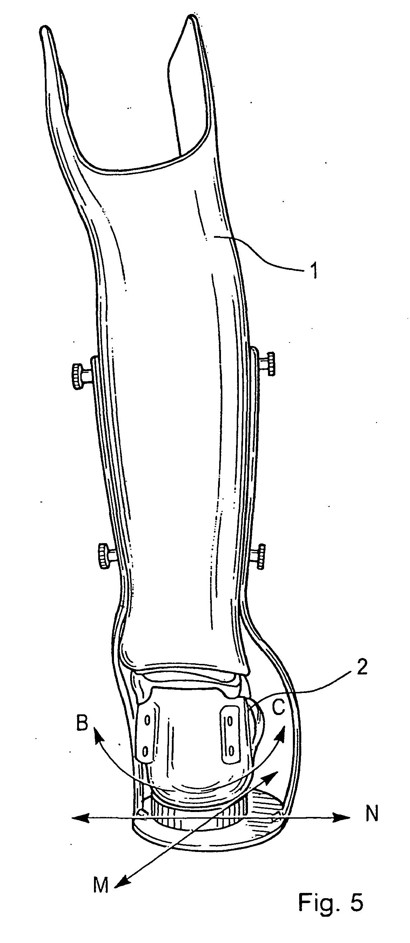

In Figur 5 ist die Orthese aus Figur 4 von hinten gezeigt. Pfeile M, N deuten an, dass die Pfanne 6 auf dem Auftrittsbügel 4 in der Transversalebene verschiebbar befestigt ist. Pfeile B und C deuten Drehungen zur Herstellung einer Pronation bzw. Supination an; die Fußschale 2 ist in der Frontalebene, also um ihre Länge drehbar.

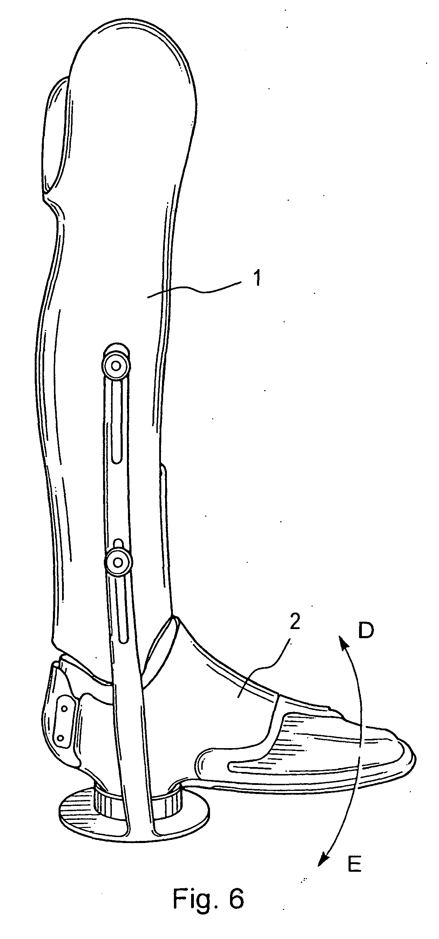

Figur 6 zeigt dieselbe Orthese von ihrer rechten Seite. Pfeile D und E deuten die Drehbarkeit der Fußschale 2 nach oben und nach unten in der Sagittalebene an.

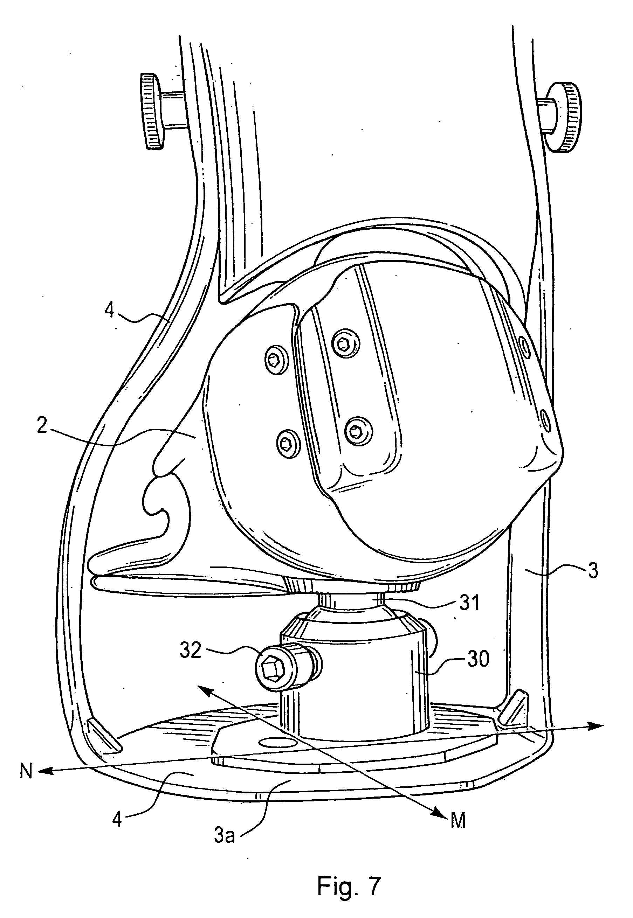

In Figur 7 ist der untere Abschnitt einer weiteren Orthese mit Kugelgelenk gezeigt. Die Fußschale 2 ist hier über ein anderes Gelenk 30 an dem Auftrittsbügel 4 abgestüzt. Auch hier ist das Gelenk 30 entlang des Auftrittsbügels 4 verschiebbar; vergleiche die Pfeile M, N. Bei dem Gelenk 30 handelt es sich um ein Kugelgelenk 30. Von dem Kugelgelenk 30 geht eine in jede Richtung kippbare Tragestange 31 ab, welche auch um ihre Längsachse drehbar ist. Fußschale 2 und Tragestange 31 sind starr miteinander verbunden. Durch die Drehbarkeit um die Längsachse der Tragestange 31 kann die Fußschale 2 in der Transversalebene gedreht werden. Durch Kippen der Tragestange 31 und Verschieben des Kugelgelenks 32 in der Transversalebene kann die Fußschale 2 auch in den anderen Ebenen gedreht werden.

An der Seite des Kugelgelenks 30 sind Schrauben 32 zu erkennen. Werden diese angezogen, so wird das Kugelgelenk 30 festgesetzt.

Figur 8 zeigt eine Orthese zur Behandlung pathologischer Deviationen in Rückfuß und zum Auseinanderziehen knöcherner Strukturen. Der obere Teil dieser Orthese mit Unterschenkelschale 1, Riemen 40, Führungsspalte 42 in Schiene 3, Führungsstiften 43 (hier nicht zu erkennen), Arbeitszylinder 50, Hebel 52, Bügel 54 und Kolbenstange 55 entspricht im wesentlichen dem Oberteil der Orthese aus Figur 2. Der untere Teil des Arbeitszylinders 50 ist, wie in Figur 2, über Kolbenstange 55 und den Bügel 54 an den Schienen 3 befestigt. Der obere Teil des Arbeitszylinders 50 ist an der Unterschenkelschale 1 befestigt (die Verbindungen zwischen dem Bügel 54 und dem Arbeitszylinder 50 beziehungsweise zwischen der Unterschenkelschale 1 und dem Arbeitszylinder 50 sind nicht explizit eingezeichnet). Weiter ist eine Fußschale 2 wie in Figur 2 vorhanden.

Der untere Abschnitt der in Figur 8 gezeigten Orthese entspricht der in den Figuren 3 und 6 gezeigten Ausführungsform. Auch hier bilden die Schienen 3 einen Auftrittsbügel 4 aus. Der Auftrittsbügel 4 ist unten zu einer Halteplatte 7 verbreitert, auf der, wie oben erläutert, eine Gelenkpfanne 6 aufgeschraubt ist. Auch hier ist die Fußschale 2 formschlüssig in der Pfanne 6 beweglich befestigt.

Die Schienen 3 weisen am unteren Ende der Unterschenkelschale 1 beziehungsweise oberhalb der Fußschale 2 ein Gelenk 70 auf, so dass der untere Teil der Schienen 3 relativ zu deren oberen Teil nach vorne und hinten verkippt werden kann. Die Drehachse korrespondiert mit dem oberen Sprunggelenk. Der Freiraum zwischen Fußschale 2 und Unterschenkelschale 1 erlaubt eine entsprechende Bewegung. Das Gelenk 70 ist lösbar und fixierbar für jeden einstellbaren Kippwinkel. Das lösbar und fixierbare Gelenk 70 kann auch einen Motor zum Kippen und Fixieren umfassen. Alternativ zu einem einen Motor aufweisenden Gelenk 70 können die Fußschale 2 und die Unterschenkelschale 1 über einen Antrieb miteinander verbunden sein, welcher über eine Stange die Fußschale 2 und die Unterschenkelschale 1 anpackt. Die Stange ist dabei beispielsweise im Vorderfußbereich der Fußschale 2 und im unteren Bereich der Unterschenkelschale 1 befestigt (nicht gezeigt).

Mit dem Gelenk 70 wird die Möglichkeit zur Stellungskorrektur erweitert. Außerdem kann über das Gelenk 70 und den ggf. zusätzlich vorhandenen Motor das Sprunggelenk mobilisiert werden. So kann beispielsweise ein Einsteifen nach einer Operation, etwa einer Klumpfußoperation, verhindert werden. Weiter kann über das Gelenk 70 und einen entsprechenden Motor auch Arthrose therapiert werden. Hier ist es besonders vorteilhaft, dass Fußschale 2 und Unterschenkelschale 1 dabei auch auseinander gezogen werden können. Dies vereinfacht die Bewegungstherapie und vermeidet auch Schmerzen.

Insgesamt ermöglicht diese Orthese sämtliche oben angeführten Bewegungen und Drehungen der Fußschale 2 relativ zu der Unterschenkelschale 1. Außerdem können bei dieser Orthese Unterschenkelschale 1 und Fußschale 2 auseinander gezogen werden. Zusätzlich kann die Fußschale 2 über das Gelenk 70 nach vorne und nach hinten verkippt werden.

### Bezugszeichenliste

- 1: Unterschenkelschale
- 2: Fußschale
- 3: seitliche Schiene
- 3a: Übergangsstück
- 4: Auftrittsbügel
- 5: Aussparung
- 6: Pfanne
- 7: Halteplatte
- 8: Fersenklappe
- 9: Innenhülle
- 10: Innenhülle
- 11: Verschraubung
- 12: Auswölbung
- 20: Auswölbung
- 30: Gelenk
- 31: Tragestange
- 32: Schraube
- 40: Riemen
- 41: Riemen
- 42: Führungsspalt
- 43: Führungsstift
- 50: Arbeitszylinder
- 51: Arretierblech
- 52: Hebel
- 53: Hebel
- 54: Bügel
- 55: Kolbenstange
- 60: Fußschalenaufhängung
- 70: Gelenk

- A - E: Drehrichtung

- M, N: Verschieberichtung

## Patentansprüche

1. Orthese mit
einer ersten Befestigungseinrichtung (1, 2) zum Fixieren der Orthese an einem Körper auf einer Seite einer flexiblen knöchernen Struktur des Körpers, insbesondere eines intraartikulären Spalts,
einer zweiten Befestigungseinrichtung (2, 1) zum Fixieren der Orthese an dem Körper auf der, in Längsrichtung der Orthese, anderen Seite der knöchernen Struktur, und
einer die erste Befestigungseinrichtung (1, 2) und die zweite Befestigungseinrichtung (2, 1) miteinander verbindenden Einstelleinrichtung (50), welche dazu ausgelegt ist, durch ihre Betätigung wahlweise einen verkürzten oder einen verlängerten Abstand zwischen der ersten und der zweiten Befestigungseinrichtung (2, 1) vor deren Fixieren am Körper herzustellen,
**dadurch gekennzeichnet, dass**
die Befestigungseinrichtungen jeweils ein anatomisch formschlüssiges Schalenteil (1, 2) zum zumindest abschnittsweise festen Umfassen des Körpers unter Abstützung an prominenten knöchernen Strukturen aufweisen und
die Einstelleinrichtung (50) dazu ausgelegt ist, durch ihre Betätigung nach dem Fixieren der beiden Schalenteile an dem Körper einen verlängerten Abstand zwischen den beiden Schalenteilen (1, 2) durch deren Auseinanderdrücken herzustellen, um die flexible knöcherne Struktur auseinanderzuziehen und gestreckt zu halten.

2. Orthese nach Anspruch 1, bei der die Einstelleinrichtung (50) dazu ausgelegt ist, frei wählbare Abstände, von einem minimalen Abstand bis zu einem maximalen Abstand, zwischen der ersten und der zweiten Befestigungseinrichtung (2, 1) herzustellen.

3. Orthese nach Anspruch 1 oder 2, bei der die erste Befestigungseinrichtung (1, 2) in Streckrichtung unbeweglich mit einer Schiene verbunden ist und die zweite Befestigungseinrichtung (2, 1) durch die Schiene geführt ist.

4. Orthese nach einem der vorangehenden Ansprüche, bei der die Einstelleinrichtung (50) eine Schaltfedereinrichtung (50) umfasst.

5. Orthese nach einem der Ansprüche 1 bis 3, bei der die Einstelleinrichtung (50) eine Gewindestange umfasst.

6. Orthese nach einem der vorangehenden Ansprüche, bei der die Einstelleinrichtung (50) dazu ausgelegt ist, den Abstand zwischen der ersten Befestigungseinrichtung (1, 2) und der zweiten Befestigungseinrichtung (2, 1) starr herzustellen.

7. Orthese nach einem der Ansprüche 1 bis 5, bei der die Einstelleinrichtung (50) dazu ausgelegt ist, den Abstand zwischen der ersten Befestigungseinrichtung (1, 2) und der zweiten Befestigungseinrichtung (2, 1) federnd-nachgiebig herzustellen.

8. Orthese nach einem der vorangehenden Ansprüche zur Streckung des Sprunggelenks, mit einer Unterschenkelschale zum Fixieren der Orthese an dem Unterschenkel durch Abstützung an dem Schienbeinplateau und den Schienbeinkondylen und
einer Fußschale (2) zum Fixieren der Orthese an dem Fuß mit zumindest abschnittweiser ringförmiger Fassung (2) für den Fuß.

9. Orthese nach einem der vorangehenden Ansprüche, insbesondere auch zur Korrektur von Gelenkfehlstellungen, mit
voneinander zumindest teilweise trennbaren Befestigungseinrichtungen (1, 2) zur Fassung der zu korrigierenden Extremität an auseinanderliegenden Stellen, wobei die betreffenden Befestigungseinrichtungen (1, 2) noch nach dem Anziehvorgang in Bezug zueinander justierbar sind und die gegenseitige Justierung durch eine leicht herstellbare Verbindung in vorgegebener gegenseitiger Position erfolgt.

10. Orthese nach einem der vorangehenden Ansprüche zur Behandlung pathologischer Deviationen im Rückfuß mit
einer oberen Fixiereinrichtung (1) zum Fixieren der Sprungbeinrolle in der Knöchelgabel und einem Fußteil (2), das zur Aufnahme der subtalaren Fußplatte eingerichtet ist und relativ zu der oberen Fixiereinrichtung (1) in der Transversalebene zwischen mindestens zwei Ausrichtungen drehbar und in jeder der Ausrichtungen lösbar fixierbar ist.

## Claims

1. An orthosis, comprising
a first fixing means (1, 2) for fixing the orthosis at a body on a side of a flexible bony structure of the body, in particular of an intra-articular gap,
a second fixing means (2, 1) for fixing the orthosis at the body on the other side of the bony structure, as seen in longitudinal direction of the orthosis, and
an adjusting means (50) connecting the first fixing means (1, 2) and the second fixing means (2, 1) with each other, which adjusting means is designed to selectively create, by its actuation, a shortened or lengthened distance between the first and the second fixing means (2, 1) before fixing the same at the body,
**characterized in that**
the fixing means each include an anatomically positive shell part (1, 2) for at least partly firmly enclosing the body by supporting on prominent bony structures, and
the adjusting means (50) is designed to create by actuating the same after fixing the two shell parts at the body a lengthened distance between the two shell parts (1, 2) by pushing them apart, in order to pull the flexible bony structure apart and keep it stretched.

2. The orthosis according to claim 1, in which the adjusting means (50) is designed to create freely selectable distances, from a minimum distance to a maximum distance, between the first and the second fixing means (2, 1).

3. The orthosis according to claim 1 or 2, in which the first fixing means (1, 2) is immovably connected with a rail in stretching direction and the second fixing means (2, 1) is guided by the rail.

4. The orthosis according to any of the preceding claims, in which the adjusting means (50) comprises a switch spring means (50).

5. The orthosis according to any of claims 1 to 3, in which the adjusting means (50) comprises a threaded rod.

6. The orthosis according to any of the preceding claims, in which the adjusting means (50) is designed to create the distance between the first fixing means (1, 2) and the second fixing means (2, 1) in a rigid manner.

7. The orthosis according to any of claims 1 to 5, in which the adjusting means (50) is designed to create the distance between the first fixing means (1, 2) and the second fixing means (2, 1) in an elastically resilient manner.

8. The orthosis according to any of the preceding claims for stretching the ankle joint, comprising a lower leg shell for fixing the orthosis at the lower leg by supporting on the tibial plateau and the tibial condyles, and
a foot shell (2) for fixing the orthosis at the foot with an at least partly ring-shaped holder (2) for the foot.

9. The orthosis according to any of the preceding claims, in particular also for the correction of joint misalignments, comprising
fixing means (1, 2) at least partly separable from each other for gripping the extremity to be corrected at points located away from each other, wherein the respective fixing means (1, 2) are adjustable relative to each other even after the dressing operation and the mutual adjustment is effected by an easily creatable connection in a specified mutual position.

10. The orthosis according to any of the preceding claims for the treatment of pathological deviations in the hindfoot, comprising
an upper fixing means (1) for fixing the talar trochlea in the mortice,and a foot part (2) which is equipped for accommodating the subtalar foot plate and is rotatable relative to the upper fixing means (1) in the transversal plane between at least two orientations and is releasably fixable in each of the orientations.

## Revendications

1. Orthèse, comprenant
un premier moyen de fixation (1, 2) pour fixer l'orthèse sur un corps sur un côté d'une structure osseuse flexible du corps, en particulier un espace interarticulaire,
un second moyen de fixation (2, 1) pour fixer l'orthèse sur le corps sur l'autre côté, en direction longitudinale de l'orthèse, de la structure osseuse, et
un moyen de réglage (50) qui relie le premier moyen de fixation (1, 2) et le second moyen de fixation (2, 1) l'un avec l'autre et qui est conçu pour établir, par son actionnement, au choix une distance raccourcie ou une distance rallongée entre le premier et le second moyen de fixation (2, 1) avant leur fixation sur le corps,
**caractérisée en ce que**
les moyens de fixation comportent chacun une partie en coque (1, 2) dont la forme établit un blocage anatomique, destinée à enserrer fermement le corps au moins par tronçon avec soutien sur des structures osseuses proéminentes, et
le moyen de réglage (50) est conçu pour établir, par son actionnement après la fixation des deux parties en coque sur le corps, une distance rallongée entre les deux parties en coque (1, 2) en les poussant en écartement, afin d'écarter la structure osseuse flexible et la tenir en situation étirée.

2. Orthèse selon la revendication 1, dans laquelle le moyen de réglage (50) est conçu pour établir des distances librement choisies, depuis une distance minimale jusqu'à une distance maximale, entre le premier et le second moyen de fixation (2, 1).

3. Orthèse selon la revendication 1 ou 2, dans laquelle le premier moyen de fixation (1, 2) est relié avec une tringle sans possibilité de déplacement en direction d'étirage, et le second moyen de fixation (2, 1) est guidé par la tringle.

4. Orthèse selon l'une des revendications précédentes, dans laquelle le moyen de réglage (50) comprend un moyen à ressort commutable (50).

5. Orthèse selon l'une des revendications 1 à 3, dans laquelle le moyen de réglage (50) comprend une tige filetée.

6. Orthèse selon l'une des revendications précédentes, dans laquelle le moyen de réglage (50) est conçu pour établir de façon rigide la distance entre le premier moyen de fixation (1, 2) et le second moyen de fixation (2, 1).

7. Orthèse selon l'une des revendications 1 à 5, dans laquelle le moyen de réglage (50) est conçu pour établir de manière souple et avec effet élastique la distance entre le premier moyen de fixation (1, 2) et le second moyen de fixation (2, 1).

8. Orthèse selon l'une des revendications précédentes, pour étirer l'articulation de la cheville, comprenant une coque de jambe destinée à fixer l'orthèse sur la jambe en la soutenant sur le plateau tibial et sur les condyles du tibia, et
une coque de pied (2) destinée à fixer l'orthèse sur le pied avec une monture (2), au moins partiellement de forme annulaire, pour le pied.

9. Orthèse selon l'une des revendications précédentes, en particulier également pour la correction de défauts de position articulaires, comprenant
des moyens de fixation (1, 2) au moins partiellement séparables l'un de l'autre pour enserrer l'extrémité à corriger à des emplacements écartés l'un de l'autre, dans laquelle les moyens de fixation (1, 2) concernés sont susceptibles d'être ajustés l'un par rapport à l'autre encore après l'opération de serrage, et l'ajustement réciproque a lieu dans la position réciproque prédéterminée grâce à une liaison aisée à établir.

10. Orthèse selon l'une des revendications précédentes, destinée au traitement de déviations pathologiques dans la partie arrière du pied, comprenant
un dispositif de fixation supérieur (1) destiné à fixer la tête de l'astragale dans la fourche malléolaire, et une partie de pied (2), qui est conçue pour recevoir le plateau plantaire sous le talus et qui est susceptible d'être fixée avec possibilité de rotation entre au moins deux orientations par rapport au moyen de fixation supérieur (1) dans le plan transversal, et détachable dans chacune des orientations.
